# EUROPEAN PATENT SPECIFICATION

(11) **EP 1 713 339 B1**
(45) Date of publication and mention of the grant of the patent: **07.11.2007**
(21) Application number: 05708315.6
(22) Date of filing: 14.02.2005
(51) Int. Cl.: A01N 65/00, A01N 27/00, A01N 25/18, A61L 2/20, A62D 3/00

(54) **HOTPLATE EMANATION**
AROMAABGABE VON EINER HEIZPLATTE
DIFFUSION D'AROMES PAR PLAQUE CHAUFFANTE

(30) Priority: 13.02.2004 GB 0403231
(43) Date of publication of application: 25.10.2006
(73) Proprietor: Reckitt Benckiser (UK) Limited, Slough, Berkshire SL1 3UH (GB); UNIVERSITY OF SOUTHAMPTON, Highfield, Southampton SO17 1BJ (GB)
(72) Inventor: HIGGINS, Sabrina, Highfield Southhampton SO17 1BJ (GB); HUGHES, John, Highfield Southhampton SO17 1BJ (GB); MCKECHNIE, Malcolm, Tom, Hull HU8 7DS (GB)
(74) Representative: Bowers, Craig Malcolm
(86) International application number: PCT/GB2005/000491
(87) International publication number: WO 2005/079583

(56) References cited:
- EP-A- 0 716 143
- EP-A- 0 843 965
- WO-A-01/76371
- WO-A-93/15774
- CA-A1- 2 222 911
- GB-A- 2 367 243
- "Product Name: Cold-Pressed Orange Oil" MATERIAL SAFETY DATA SHEET, [Online] January 2003 (2003-01), XP002332677 Retrieved from the Internet: URL:http://www.pdmchemicals.com/MSDS/MSDS- Orange%20Oil.pdf> [retrieved on 2005-06-20]
- S.BUDAVARI (ED.): "The Merck Index" 1989, MERCK & CO. INC. , RAHWAY, N.J., USA , XP002332678 entry 7415. "beta-Pinene"
- DATABASE WPI Section Ch, Week 200367 Derwent Publications Ltd., London, GB; Class D16, AN 2003-701561 XP002332679 & JP 2003 180865 A (MITSUBISHI JUKOGYO KK) 2 July 2003 (2003-07-02)

## Description

The present invention relates to a method of deactivating dust mite allergens.

Various allergens are known to trigger a human reaction. For example, it has been known for a long time that house dust can trigger allergenic reactions in humans, such as asthma and rhinitis. It was reported, as early as 1928 that it was the dust mites in the dust that were the primary source of the allergenic response, but it was only in the 1960's that researchers appreciated its significance.

House dust mites produce detritus which causes allergenic reaction in many people. The major allergens are believed to be detritus from the mite species Dermatophogoides farinae and Dermatophagoides pteronyssinus (the allergens being known as Der fl and Der p1 respectively). The detritus includes faeces as well as body part residues of the mites. A review is given in Experimental and Applied Acarology, 10 (1991) p. 167-186.

WO99/15208 describes a method for deactivating allergens derived from the D. Pteronyssinus and D. Farinae dust mite species, which comprises contracting the allergen with one of 28 deactivants which are described. These may be delivered into an airspace by aerosol spraying.

WO 01/76371 describes further deactivants for house dust mite allergens. These may be delivered into an airspace by various methods including use of an ultra-sonic jet nebuliser, an ion wind generator, a candle incorporating a deactivant, or heat to vaporise an oil. The examples given of the use of heat are to float the oil on water in an oil burner or to heat oil directly in an oil burner. Alternatively the volatile oil may be vaporized from a heated wick dipped into a reservoir of the volatile oil.

In accordance with a first aspect of the present invention there is provided a method of deactivating Der p and Der f allergens, the method comprising dispersing into an airspace an allergen-deactivating amount of an allergen-deactivating compound (hereinafter the "deactivant"), the deactivant being dispersed from a vessel in contact with a hotplate, the hotplate preferably being set to a temperature of at least 100°C.

Use of the noun deactivant and the verb deactivate in this specification denote that some or all of a source of allergens at a locus are rendered unable to evoke an allergenic response in a human, by a method of the present invention. The net result is that the source may be reduced in its allergenicity, or its allergenicity may be completely removed.

Many methods of killing dust mites are known. However it is thought that the allergenic response is due to dust mite detritus, not the dust mites themselves. Thus killing them does not remove the detritus. In the method of the present invention, the allergens Der p and/or Der f are deactivated. The killing of dust mites is not necessary.

The use of a hotplate enables the heat applied to disperse the deactivant to be controlled, in a manner which is not possible with prior methods.

Preferably the deactivant is dispersed into the airspace as a vapour.

Our work suggests that use of a hotplate below 100°C gives some allergen deactivating activity but that use of a higher temperature gives allergen deactivating activity of a substantially and surprisingly higher level, even though the quantity of deactivant dispersed may be the same in each case.

Preferably the hotplate has an electrical heat source.

Preferably the vessel and the hotplate are in face-to-face contact. Preferably the hotplate has a flat surface and the vessel has a flat base, and the vessel rests on the hotplate. Preferably the vessel has an opening in its upper region. Preferably it has a fully open upper face. Preferably, therefore, the vessel has a flat base, a side (if cylindrical) or sides depending upwardly therefrom, and no further side.

Preferably the hotplate is set to a temperature of at least 130°C.

Preferably the hotplate is set to a temperature up to 300°C, preferably up to 250°C.

Preferably the deactivant is selected from:
a terpene hydrocarbon;
a citrus oil;
a mint oil;
bois de rose oil;
oil of jasmine;
frankincense;
oil of bergamot; and
oil of lemon grass.

Preferred terpene hydrocarbons include tea tree oil, pinol and β-pinene.

An especially preferred deactivant is a citrus oil, most preferably orange oil.

Another especially preferred deactivant is β-pinene.

A deactivant may suitably be a single compound. Alternatively a mixture of deactivants may be used together.

A deactivant may be part of a blend of compounds, not all of which are deactivants. For example a citrus oil is a blend of compounds not all of which will function as deactivants.

A deactivant may suitably be dispersed into the airspace over an extended period, for example at least 30 minutes, and preferably at least 1 hour.

A deactivant may suitably be dispersed into the airspace on two occasions, interrupted by a period in which there is no deactivant dispersal. A deactivant may be dispersed into the airspace on one or more further occasions, following a corresponding period or periods of no deactivant dispersal. Preferably each such dispersal occasion involves deactivant dispersal over an extended period, as described above. Preferably the or each period in which there is no deactivant dispersal is an extended period, for example at least 2 hours, preferably at least 4 hours, and most preferably at least 8 hours. We have found that the method produces a prolonged reduction in the allergen loading of an allergen-contaminated inanimate substrate. Delivery of the deactivant into an airspace as described causes a permanent reduction in the population of allergens in an inanimate test source. By inanimate test source we mean a test source which is itself inanimate (e.g. it is not the skin or coat/fur of a live animal) and it does not contain living organisms, such as dust mites. Populations of dust mites would make any result difficult to interpret.

We have found that the reduction in allergen content in such a source is of long duration, for example at least 7 days, typically at least 14 days, and suitably at least 28 days. Indeed, in tests we have carried out over a 28-day period, we have found that the allergen content may continue to decline over time, even though the deactivant may have been used days or weeks before. The results suggest that the allergenic species have been truly denatured or degraded, to the extent that, firstly, they cannot re-form, and secondly, their degradation products are not themselves allergenic. It further suggests that the action of the deactivant is not merely a masking or damping effect. Any such effect would be likely to break down over time.

The deactivant may be used as such, or may be comprised within an oil-on-water formulation, or may be comprised within an oil-in-water emulsion formulation. An oil/water formulation suitably comprises at least 0.5% by weight of the deactivant (in total, when more than one of said deactivants is employed), preferably at least 2%, more preferably at least 6%, most preferably at least 8%, and especially at least 10%. Suitably an oil-water formulation comprises up to 25% by weight of the deactivant (in total, when more than one of said deactivants is employed), more preferably up to 20% and most preferably up to 15%.

In this specification unless otherwise stated a percentage value given for a component denotes the weight of the component expressed as a percentage of the total weight of the emulsion.

The formation of emulsions is generally well known in the art and is described, for example, in Modern Aspects of Emulsion Science, edited by Bernard P. Binks, The Royal Society of Chemistry, 1998 and Surfactant Science and Technology, Second Edition, Drew Myers, 1992, VCH Publishers, Inc. Non-ionic surfactants may be especially suitable. Proprietary surfactant packs may be employed to form emulsions, for example E-Z-MULSE (Trade Mark), a non-ionic surfactant pack from Florida Chemical Company, US.

The present invention involves the dispersal of an allergen deactivant into an airspace, preferably as a vapour. It is possible that airborne allergens may be deactivated but it is believed that there is effective deactivation of allergens borne on surfaces within the airspace.

In accordance with a further aspect of the present invention there is provided the use of apparatus for deactivating Der p and/or Der f allergens at a locus, the apparatus comprising an allergen deactivant within a vessel, and a heat source used to accelerate the vaporization of the deactivant, the heat source being a hotplate in contact with the vessel, the hotplate being at a temperature of at least 100°C.

In accordance with a further aspect there is provided an apparatus for deactivating Der p and/or Der f allergens comprising a vessel containing an allergen deactivant and a hot plate in contact with the vessel, the hotplate being capable of being set to a temperature of at least 100°C.

The present invention will be further described with reference to the following Examples.

### Experimental Protocol

When using house dust for allergen denaturing tests an inherent difficulty is the variability of the amount of allergen in each small sample, even when taken from the same dust reservoir. The amount of dust in the pre-treatment sample must be accurately estimated in order to determine the extent of any allergen denaturing. In these tests the dust sample was applied to the test exposure surface and then one half of this surface dust was removed to measure the control pre-treatment allergen level of that specific sample. Each control was directly relevant to each sample, which gave the best possible estimate of the level of allergen in the sample before exposure to possible denaturant. All tests employed a glass reinforced plastic booth of size 0.7m x 0.7m x 1.0m. Average values are stated.

The following Examples all measure the reduction of the house dust mite Dermatophagoides pteronyssinus allergen - Der p1.

House dust was passed through a number of sieves and the fraction smaller than 53 µm was collected. 0.025g of dust was placed in a small sieve to distribute it evenly over the test surface. The test surface was a PTFE (polytetrafluoroethylene - trade mark TEFLON) coated metal tray of size 30cm x 30cm. The dust was applied to the tray by moving the sieve continuously over the surface while tapping the sieve. One half of the dust was then removed by suction onto an in-line filter and the weight recorded, this was the pre-treatment control.

The tray was then placed in the booth. Three tea light holders - upwardly open cylindrical vessels (diameter 40mm, height 15mm) produced to hold nightlight candles - each containing 6ml of water and 0.8ml of orange oil - were placed together on an electric hotplate set to 250°C. In practice we found that this meant that the hotplate temperature cycled between 130°C and 250°C.

Three more tea light holders each containing 6.8ml of 12% orange oil/water emulsified with E-Z-MULSE were placed together on an electric hotplate set to 250°C. In practice we found that this meant that the hotplate temperature cycled between 130°C and 250°C. Heating was stopped after 105 minutes. These compositions did not evaporate to dryness. This is believed to be due to remaining non-volatile surfactant from the E-Z-MULSE constituent).

Three more tea light holders each containing 6ml of water and 0.8ml of orange oil were placed on the hotplate, set to 90°C. In practice we found that this meant that the hotplate temperature cycled between 60°C and 90°C.

Three more tea light holders each containing 6ml of water and 0.8ml of orange oil were placed on the hotplate, set to 74°C. In practice we found that this meant that the temperature cycled between 40°C and 74°C.

Three more tea light holders each containing 6ml of water and 0.8ml of orange oil were placed on the hotplate, set to 50°C. In practice we found that this meant that the hotplate temperature cycled between 30°C and 50°C.

Three more tea light holders each containing 6ml of water and 0.8ml of orange oil were placed on the hotplate, set to 25°C. In practice we found that this meant the hotplate temperature cycled between 20°C and 25°C.

As a further comparison three tea lights containing only water (6.8ml each) were tested on the hotplate, set to 74°C. In practice we found that this meant the temperature cycled between 40°C and 74°C.

For each test the booth was sealed. Heat was delivered for sufficient time to allow the liquid to fully evaporate and then the hotplate was allowed to cool. After 24 hours the tray was removed, the dust was collected from it and its weight recorded. The booth was washed with strong detergent between tests.

The test samples were assayed for the Der p1 allergen using an ELISA (Enzyme linked immunosorbent assay) to determine the allergen content. This was then related to the weight of dust that had been present in each sample. All of the samples were multiplied up to compare the amount of allergen expected to be present in a 0.1g sample of dust. The percentage difference between the control sample and the exposed sample was then obtained.

The Der p1 allergen reductions were as follows:
Hot plate set to 130-250°C, 0.8ml orange oil on 6ml water - 75.4%
Hot plate set to 130-250°C, 0.8ml orange oil and 6ml water, emulsified - 91.0%
Hot plate set to 60-90°C, 0.8ml orange oil on 6ml water - 48.6%
Hot plate set to 40-74°C, 0.8ml orange oil on 6ml water - 47.9%
Hot plate set to 30-50°C, 0.8ml orange oil on 6ml water - 48.0%
Hot plate set to 20-25°C, 0.8ml orange oil on 6ml water - 44.6%
Hot plate set to 40-74°C, 6.8ml water only - 42.7%

In the tests at 20-25°C there was incomplete evaporation.

Statistical analysis suggested that the finding of increased activity at a hotplate temperature of 130-250°C was a significant result, compared with activity at 60-90°C.

It was found that the allergen content did not substantially recover over time.

## Claims

1. A method of deactivating Der p and/or Der f allergens, the method comprising dispersing into an airspace an allergen-deactivating amount of an allergen-deactivating compound (hereinafter the "deactivant"), the deactivant being dispersed as a vapour from a vessel in contact with a hotplate, the hotplate being at a temperature of at least 100°C.

2. A method as claimed in claim 1, wherein the hotplate is at a temperature of at least 130°C.

3. A methods as claimed in claim 1 or 2, wherein the hotplate is at a temperature of up to 300°C.

4. A method as claimed in claim 3, wherein the hotplate is at a temperature of up to 250°C.

5. A method as claimed in any preceding claim, wherein the deactivant is dispersed into the airspace over at least 30 minutes.

6. A method as claimed in any preceding claim, wherein the deactivant is selected from:
a terpene hydrocarbon;
a citrus oil;
a mint oil;
bois de rose oil;
oil of jasmine;
frankincense;
oil of bergamot;
oil of lemon grass;
or a component thereof.

7. A method as claimed in any preceding claim, wherein the deactivant comprises a terpene hydrocarbon.

8. A method as claimed in any preceding claim, wherein the deactivant comprises β-pinene.

9. A method as claimed in any preceding claim, wherein the deactivant comprises orange oil or a component thereof.

10. A method as claimed in any preceding claim, wherein the vessel is an upwardly open vessel.

11. The use of apparatus for deactivating Der p and/or Der f allergen at a locus, the apparatus comprising an allergen deactivant within a vessel, and a heat source used to accelerate the vaporization of the deactivant, the heat source being a hotplate in contact with the vessel, the hotplate being at a temperature of at least 100°C.

12. An apparatus for deactivating Der p and/or Der f allergens comprising a vessel containing an allergen deactivant and a hotplate in contact with the vessel, the hotplate being capable of being set to a temperature of at least 100°C.

## Patentansprüche

1. Verfahren zum Deaktivieren von Der-p- und/oder Der-f-Allergenen, wobei das Verfahren das Verteilen einer Allergene reduzierenden Menge einer Allergene deaktivierenden Verbindung (hier nachstehend das "Deaktivierungsmittel") in einem Luftraum umfasst, wobei das Deaktivierungsmittel als Dampf aus einem Gefäß in Kontakt mit einer Heizplatte verteilt wird, wobei die Heizplatte eine Temperatur von mindestens 100°C aufweist.

2. Verfahren nach Anspruch 1, wobei die Heizplatte eine Temperatur von mindestens 130°C aufweist.

3. Verfahren nach Anspruch 1 oder 2, wobei die Heizplatte eine Temperatur von bis zu 300°C aufweist.

4. Verfahren nach Anspruch 3, wobei die Heizplatte eine Temperatur von bis zu 250°C aufweist.

5. Verfahren nach einem der vorangehenden Ansprüche, wobei das Deaktivierungsmittel über eine Dauer von mindestens 30 Minuten im Luftraum verteilt wird.

6. Verfahren nach einem der vorangehenden Ansprüche, wobei das deaktivierungsmittel ausgewählt ist aus:
einem Terpenkohlenwasserstoff;
einem Zitrusöl;
einem Pfefferminzöl
Rosenholzöl;
Jasminöl;
Weihrauch;
Bergamotteöl; und
Zitronengrasöl;
oder einem Bestandteil davon.

7. Verfahren nach einem der vorangehenden Ansprüche, wobei das Deaktivierungsmittel einen Terpenkohlenwasserstoff umfasst.

8. Verfahren nach einem der vorangehenden Ansprüche, wobei das Deaktivierungsmittel β-Pinen umfasst.

9. Verfahren nach einem der vorangehenden Ansprüche, wobei das Deaktivierungsmittel Orangenöl oder einen Bestandteil davon umfasst.

10. Verfahren nach einem der vorangehenden Ansprüche, wobei das Gefäß ein nach oben offenes Gefäß ist.

11. Verwendung eines Geräts zum Deaktivieren von Der-p- und/oder Der-f-Allergenen an einem Ort, wobei das Gerät ein Allergendeaktivierungsmittel in einem Gefäß und eine zum Beschleunigen der Verdampfung des Deaktivierungsmittels verwendete Heizquelle umfasst, wobei die Heizquelle eine Heizplatte in Kontakt mit dem Gefäß ist, wobei die Heizplatte eine Temperatur von mindestens 100°C aufweist.

12. Gerät zum Deaktivieren von Der-p- und/oder Der-f-Allergenen, umfassend ein Gefäß, das ein Allergendeaktivierungsmittel und eine Heizplatte in Kontakt mit dem Gefäß enthält, wobei die Heizplatte auf eine Temperatur von mindestens 100°C eingestellt werden kann.

## Revendications

1. Procédé de désactivation d'allergènes Der p et/ou Der f, procédé comprenant la dispersion dans un espace d'air d'une quantité désactivatrice d'allergènes d'un composé désactivateur d'allergènes (ci-après le "désactivant"), le désactivant étant dispersé sous forme de vapeur à partir d'un récipient en contact avec une plaque chauffante, la plaque chauffante étant à une température d'au moins 100°C.

2. Procédé suivant la revendication 1, dans lequel la plaque chauffante est à une température d'au moins 130°C.

3. Procédé suivant la revendication 1 ou 2, dans lequel la plaque chauffante est à une température allant jusqu'à 300°C.

4. Procédé suivant la revendication 3, dans lequel la plaque chauffante est à une température allant jusqu'à 250°C.

5. Procédé suivant l'une quelconque des revendications précédentes, dans lequel le désactivant est dispersé dans l'espace d'air pendant au moins 30 minutes.

6. Procédé suivant l'une quelconque des revendications précédentes, dans lequel le désactivant est choisi entre :
un hydrocarbure terpénique ;
une essence d'agrume ;
une essence de menthe ;
une essence de bois de rose ;
une essence de jasmin ;
l'encens ;
l'essence de bergamote ;
l'essence de citronnelle ;
ou un de leurs constituants.

7. Procédé suivant l'une quelconque des revendications précédentes, dans lequel le désactivant comprend un hydrocarbure terpénique.

8. Procédé suivant l'une quelconque des revendications précédentes, dans lequel le désactivant comprend du β-pinène.

9. Procédé suivant l'une quelconque des revendications précédentes, dans lequel le désactivant comprend de l'essence d'orange ou un de ses constituants.

10. Procédé suivant l'une quelconque des revendications précédentes, dans lequel le récipient est un récipient ouvert vers le haut.

11. Utilisation d'un appareil pour désactiver les allergènes Der p et/ou Der f dans un milieu, appareil comprenant un désactivant d'allergènes dans un récipient, et une source de chaleur utilisée pour accélérer la vaporisation du désactivant, la source de chaleur étant une plaque chauffante en contact avec le récipient, la plaque chauffante étant à une température d'au moins 100°C.

12. Appareil pour désactiver les allergènes Der p et/ou Der f, comprenant un récipient contenant un désactivant d'allergènes, et une plaque chauffante en contact avec le récipient, la plaque chauffante pouvant être portée à une température d'au moins 100°C.
